# EUROPEAN PATENT APPLICATION

(11) **EP 4 653 523 A1**
(43) Date of publication of application: **26.11.2025**
(21) Application number: 24744748.5
(22) Date of filing: 19.01.2024
(51) Int. Cl.: C12N 5/0789, A61K 35/28, A61P 7/00, A61P 7/06, A61P 35/02, C12N 5/02

(54) **CULTURE MEDIUM, PRODUCTION METHOD, AND CELL PREPARATION**

(30) Priority: 20.01.2023 JP 2023007156
(71) Applicant: National University Corporation Kumamoto University, Kumamoto 860-8555 (JP)
(72) Inventor: UMEMOTO Terumasa, Kumamoto-shi, Kumamoto 860-8555 (JP)
(74) Representative: Bryn Aarflot AS
(86) International application number: PCT/JP2024/001499
(87) International publication number: WO 2024/154828

(57) **Abstract**

There is provided a culture medium for expansion culture of a mammalian hematopoietic stem cell, where the culture medium contains a glutamate dehydrogenase inhibitor as an active ingredient.

## Description

### TECHNICAL FIELD

The present invention relates to a culture medium, a production method, and a cell preparation.

### BACKGROUND ART

Hematopoietic stem cells, which are the source of blood cells, are usually maintained in a quiescent state in the bone marrow. However, in a case where the bone marrow hematopoietic tissue is damaged by a treatment with an anti-cancer agent, irradiation with radioactive rays, or the like, the hematopoietic stem cells lead the reconstruction of the bone marrow hematopoietic tissue by using self-replication ability and pluripotency. Since hematopoietic stem cells are used for the medical treatment (hematopoietic stem cell transplantation) of leukemia, aplastic anemia, and the like, studies have been carried out worldwide to attempt to maintain and proliferate the hematopoietic stem cells in vitro.

In a case where hematopoietic stem cells are cultured under normal conditions, most of the cells are differentiated, or the activity of stem cells is significantly weakened, which makes it difficult to amplify stem cells of a transplantation therapy grade.

As a method of subjecting hematopoietic stem cells to expansion culture, for example, the method described in Patent Document 1 has been proposed.

### Citation List

### Patent Document

Patent Document 1: Japanese Unexamined Patent Application, First Publication No. 2022-113722

### SUMMARY OF INVENTION

### Technical Problem

In the method described in Patent Document 1, the amplification efficiency of hematopoietic stem cells is increased by the addition of the demethylating agent; however, the demethylating agent affects epigenetics, and thus there is room for improvement from the viewpoint of safety.

At present, it is still difficult to efficiently maintain, proliferate, and differentiate hematopoietic stem cells in vitro from the viewpoints of safety, reproducibility, efficiency, cost, and the like.

Therefore, an object of the present invention is to provide, based on a new mechanism of action, a culture medium that enables the expansion culture of hematopoietic stem cells, a production method for hematopoietic stem cells using the culture medium, and a cell preparation.

### Solution to Problem

The present invention is as follows.
[1] A culture medium for expansion culture of a mammalian hematopoietic stem cell, the culture medium containing:
   a glutamate dehydrogenase inhibitor as an active ingredient.
[2] The culture medium according to [1], in which the glutamate dehydrogenase inhibitor is R162 or a derivative thereof.
[3] A production method for a hematopoietic stem cell, in which a mammalian hematopoietic stem cell is subjected to expansion culture in a culture medium containing, as an active ingredient, a glutamate dehydrogenase inhibitor.
[4] The production method according to [3], in which the glutamate dehydrogenase inhibitor is R162 or a derivative thereof.
[5] A cell preparation containing:
   a mammalian hematopoietic stem cell in which glutamate dehydrogenase is inactivated or deficient.
[6] The cell preparation according to [5], in which in the hematopoietic stem cell, EPCR or a hematopoietic stem cell marker molecule equivalent to the EPCR is highly expressed.

### Advantageous Effects of Invention

According to the present invention, hematopoietic stem cells can be subjected to expansion culture based on a new mechanism of action.

### BRIEF DESCRIPTION OF DRAWINGS

[FIG. 1] Results obtained by analyzing a proportion of EPCR-strongly positive cells among hematopoietic stem cells isolated from a mouse, by using flow cytometry.
[FIG. 2] Results obtained by transplanting each cell group into a recipient mouse and examining a proportion of the transplanted cells after 20 weeks.
[FIG. 3] Results obtained by analyzing a proportion of EPCR-strongly positive cells using flow cytometry after ex vivo culturing of each cell group.
[FIG. 4] Results obtained by measuring the level of glutamic acid in each cell.
[FIG. 5] (A) is a diagram of a TCA cycle. (B) is a diagram of the α-ketoglutaric acid production pathway.
[FIG. 6] Results obtained by analyzing a proportion of EPCR-positive cells using flow cytometry after ex vivo culturing of the cell group under each condition.
[FIG. 7] (A) shows the quantification results of FIG. 6. (B) shows the results of the ratio of the increase in the total number of cells after ex vivo culturing of the cell group under each condition.
[FIG. 8] Results obtained by transplanting each cell group into a recipient mouse and examining the proportion of cells derived from the transplanted cells in the peripheral blood 4 weeks later (A) and 12 weeks later (B).
[FIG. 9] A flow of adjustment of a cell group that is used for the analysis of the gene expression pattern.
[FIG. 10] Results of the analysis of the gene expression pattern of each cell group. It shows the results obtained by carrying out a comprehensive gene expression analysis with RNA-sequence by using the cells acquired in the adjustment flow shown in FIG. 9 and carrying out a principal component analysis using the data of the comprehensive gene expression analysis.
[FIG. 11] Results of the analysis of the chromatin status of each cell group. It shows the results obtained by carrying out a principal component analysis by using the cells acquired in the adjustment flow shown in FIG. 9 to comprehensively acquire the information on the chromatin open regions in a genome-wide manner with ATAC-sequence and then extracting a region corresponding to a cis-element.
[FIG. 12] Results obtained by transplanting each cell group into a recipient mouse and examining the proportion of cells derived from the transplanted cells in the peripheral blood and the bone marrow after 20 weeks.
[FIG. 13] Results obtained by analyzing the proportion of CD34+, CD38-, and CD90+ cells using flow cytometry after culturing human CD34⁺ bone marrow cells in the presence of R162. Control indicates the analysis results after culturing human CD34⁺ bone marrow cells in the absence of R162.
[FIG. 14] (A) shows the results of the ratio of the increase in the total number of cells in the cell group in FIG. 13. (B) is the quantification results of FIG. 13.
[FIG. 15] (A) shows the results of analyzing the proportion of EPCR-positive cells after culturing mouse EPCR-positive cells in the presence of EGCG, by using flow cytometry. Control indicates the results in the mouse EPCR-positive cells cultured in the absence of EGCG. (B) is quantification results of (A).

### DESCRIPTION OF EMBODIMENTS

### Culture medium

In one embodiment, the present invention provides a culture medium for expansion culture of a mammalian hematopoietic stem cell, where the culture medium contains a glutamate dehydrogenase inhibitor as an active ingredient.

As described in examples later, the inventors have found that the stem cells that undergo self-replication and division in a phase of recovery of hematopoiesis in the bone marrow are derived from stem cells (EPCR High stem cells) that more strongly express, mainly in a steady state, endothelial protein C receptor (EPCR) which is a stem cell marker.

In fact, the EPCR High stem cells exhibit, in accordance with the chromatin dynamics thereof, a long-term bone marrow reconstruction ability higher than that of the EPCR Low stem cells, and relatively maintain the stem cell-like phenotype under normal culture conditions; however, a large number of the EPCR Low stem cells immediately lose the expression system of the stem cells and differentiate into progenitor cells. In addition, it has been found that a large number of the EPCR High stem cells (self-replication type stem cells) that maintained the expression system of the stem cells under the normal culture conditions also lost the EPCR High phenotype in the course of the culture, and thus the ability to act as stem cells for a long term was significantly weakened substantially.

The inventors focused on the fact that, in a case where the self-replication type stem cells in a phase of recovery of hematopoiesis in the bone marrow divides while maintaining an EPCR High phenotype, the self-replication type stem cells are independent of glutamine (Gln) unlike a case of inducing division under the normal culture conditions, and have found that, in a case where the EPCR High stem cells are cultured under a low Gln condition, the EPCR High stem cells are maintained more favorably than under the normal culture conditions.

Further, the inventors focused on glutamate dehydrogenase involved in the catabolism of glutamic acid to α-ketoglutaric acid (αKG), and have found that the inhibition of the glutamate dehydrogenase under the normal culture conditions contributes to the maintenance of the EPCR High phenotype.

The basal medium is not particularly limited, and a basal medium that is generally used for animal culture can be used. Examples of the basal medium include an IMDM medium, a Medium199 medium, an Eagle's minimum essential medium (EMEM), an αMEM medium, a Dulbecco's modified Eagle's medium (DMEM), a Ham's F12 (F12) medium, an RPMI 1640 medium, a Fischer's medium, and a mixed culture medium thereof.

The culture medium may contain serum (for example, fetal bovine serum (FBS)) or may be serum-free. As necessary, the culture medium may contain serum substitutes such as albumin, transferrin, a KnockOut Serum Replacement (KSR) (a serum substitute in a case of culturing ES cells) (Invitrogen), an N2 supplement (Invitrogen), a B27 supplement (Invitrogen), fatty acid, insulin, a collagen precursor, trace elements, 2-mercaptoethanol, and 3'-thiol glycerol. In addition, the culture medium may contain substances such as a lipid, an amino acid, L-glutamine, GlutaMAX (Invitrogen), a non-essential amino acid (NEAA), a vitamin, a growth factor, an antibiotic, an antioxidant, pyruvic acid, a buffering agent, inorganic salts, and equivalents thereof.

In the present embodiment, examples of the glutamate dehydrogenase inhibitor include epigallo catechin-3-gallate (EGCG), 3-carboxy-5-bromofuranic acid, 2-allyl-1-hydroxy-9,10-anthraquinone (R162), and derivatives thereof, and R162 or a derivative of R162 having the function equivalent to that of R162 is preferable.

As described in examples later, in a case of carrying out culturing in the presence of R162, the proportion of EPCR High stem cells can be maintained at a high level without significantly impairing the overall proliferation of stem cells as compared with the normal culture conditions. Further, the stem cells maintained by the R162 treatment exhibited a gene expression pattern that was substantially equivalent to that of the EPCR High stem cells maintained under the normal culture conditions, and they exhibited a long-term bone marrow reconstruction ability (stem cell activity) equivalent to or higher than that of the EPCR High stem cells maintained under the normal culture conditions. That is, the EPCR High phenotype can be maintained under the R162 treatment, and the cells can be amplified as EPCR High stem cells.

Examples of the mammal from which the hematopoietic stem cells are derived include a human, a cat, a dog, a horse, a monkey, a cow, a sheep, a pig, a goat, a rabbit, a hamster, a guinea pig, a rat, and a mouse, where a human or a mouse is preferable, and a human is more preferable.

### Production method for hematopoietic stem cell

In one embodiment, the present invention provides a production method for a hematopoietic stem cell, in which a mammalian hematopoietic stem cell is subjected to expansion culture in a culture medium containing, as an active ingredient, a glutamate dehydrogenase inhibitor. The glutamate dehydrogenase inhibitor is preferably R162.

The term "expansion culture" means a culture in which hematopoietic stem cells are proliferated while maintaining the stem cell-like phenotype and the self-replication (bone marrow reconstruction) ability. In the present embodiment, the culture method is not particularly limited. The hematopoietic stem cells can be cultured under culture conditions that are generally used for culturing animal cells. The culture temperature can usually be set to 25°C to 40°C, and it is preferably 30°C to 40°C. Specific examples of the culture temperature include about 37°C. The hematopoietic stem cells can be cultured in a normal atmosphere of CO₂-containing air. The CO₂ concentration can usually be set to about 0.3% to 5% and it is preferably about 2% to 5%. Specific examples of the CO₂ concentration include about 5%.

The culture period is not particularly limited and can be set to any period. In the method according to the present embodiment, by using the above-described culture medium, the long-term culture can be carried out while maintaining the phenotype of the stem cells. In a case of carrying out culturing for a long period of time, it is preferable to carry out subculture appropriately. By repeating the subculture using the above-described culture medium, it is possible to continue the culture of hematopoietic stem cells while maintaining the phenotype of the stem cells. The subculture can be carried out by collecting hematopoietic stem cells from a culture solution and seeding the hematopoietic stem cells in a new culture medium. The interval of subculturing is not particularly limited; however, it can be set to, for example, about 2 to 10 days or about 3 to 7 days.

In the method according to the present embodiment, hematopoietic stem cells are cultured using the above-described culture medium, and thus hematopoietic stem cells can be efficiently proliferated while maintaining the phenotype of the stem cells. The hematopoietic stem cells subjected to expansion culture by the method according to the present embodiment can be used for bone marrow transplantation or gene therapy.

### Cell preparation

In one embodiment, the present invention provides a cell preparation containing a mammalian hematopoietic stem cell in which glutamate dehydrogenase is inactivated or deficient. The mammalian hematopoietic stem cell that is used for the cell preparation is preferably a human hematopoietic stem cell, and more preferably a human hematopoietic stem cell derived from umbilical cord blood or a human hematopoietic stem cell derived from bone marrow. It is preferable that in the hematopoietic stem cell, EPCR or a hematopoietic stem cell marker molecule equivalent to the EPCR is highly expressed. The hematopoietic stem cell marker molecule equivalent to EPCR means a hematopoietic stem cell marker molecule of which the expression is controlled by a mechanism similar to that of EPCR.

The hematopoietic stem cell in which the glutamate dehydrogenase is inactivated or deficient refers to a hematopoietic stem cell which is cultured in the presence of a glutamate dehydrogenase inhibitor or a hematopoietic stem cell in which the expression of the glutamate dehydrogenase gene is suppressed or lost. The suppression of the expression of the glutamate dehydrogenase gene can be caused by the gene knockdown or the like by introducing, into hematopoietic stem cells, a nucleic acid sequence that causes the expression of an RNAi-inducible nucleic acid, an antisense nucleic acid, an aptamer, a ribozyme, or the like with respect to the glutamate dehydrogenase gene. The loss of the expression of the glutamate dehydrogenase gene can be caused, for example, by introducing a mutation into the glutamate dehydrogenase gene to destroy the glutamate dehydrogenase gene.

The mutation can be caused by deletion, substitution, insertion of any sequence, or the like, regarding the glutamate dehydrogenase gene or a part or whole of a gene expression regulatory region. The introduction of the mutation thereof can be carried out by using, for example, a treatment with a mutagenic substance, irradiation with ultraviolet rays, gene targeting by a homologous recombination technology or the like, gene knockout, conditional knockout, or the like. In addition, a genome editing technology may be used for the gene targeting and the gene knockout.In this manner, the produced cell preparation is administered to a patient by intravenous injection or the like. Examples

Hereinafter, the present invention will be described in more detail with reference to experimental examples; however, the present invention is not limited to these examples.

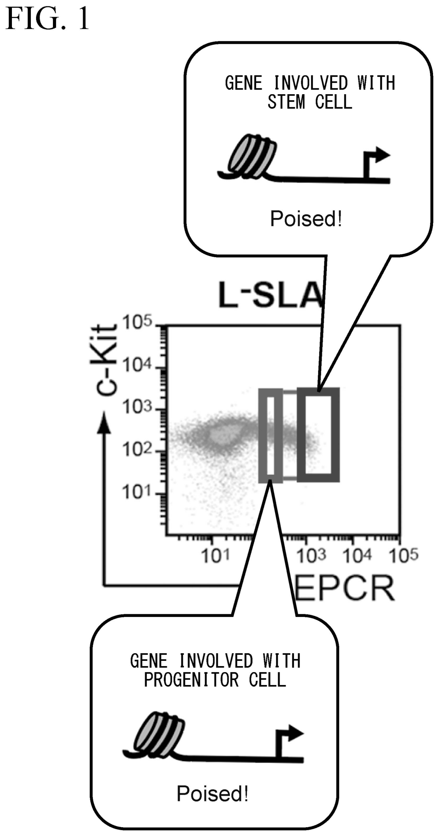

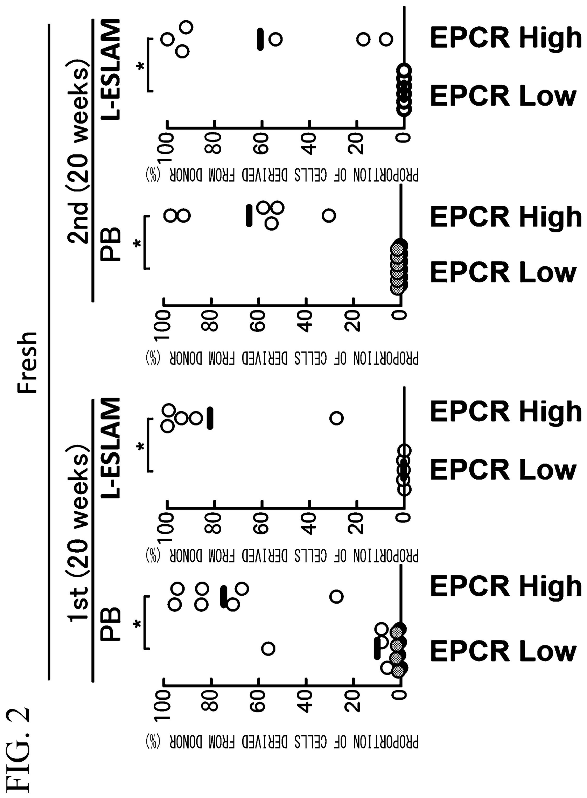

The expression of c-kit and EPCR in a hematopoietic stem cell fraction (lineage: CD150+, CD48-) isolated from a mouse by using flow cytometry was checked. Among the c-kit-positive and EPCR-positive cells, a cell group of the top 30% of the EPCR-positive cells was defined as an EPCR High cell group, and a cell group of the bottom 30% of the EPCR-positive cells was defined as an EPCR Low cell group (see FIG. 1). Each cell group was transplanted into a recipient mouse, and 20 weeks later, the proportion of cells derived from the transplanted stem cells was examined in the peripheral blood (PB). In addition, at the same time, the proportion of cells derived from the transplanted stem cells was examined in the hematopoietic stem cell fraction (L-ESLAM) in the bone marrow after 20 weeks of the transplantation. The experiment was carried out twice. As shown in FIG. 2, it was confirmed that the EPCR-positive cell group undergoes self-replication and division in the body of the host.

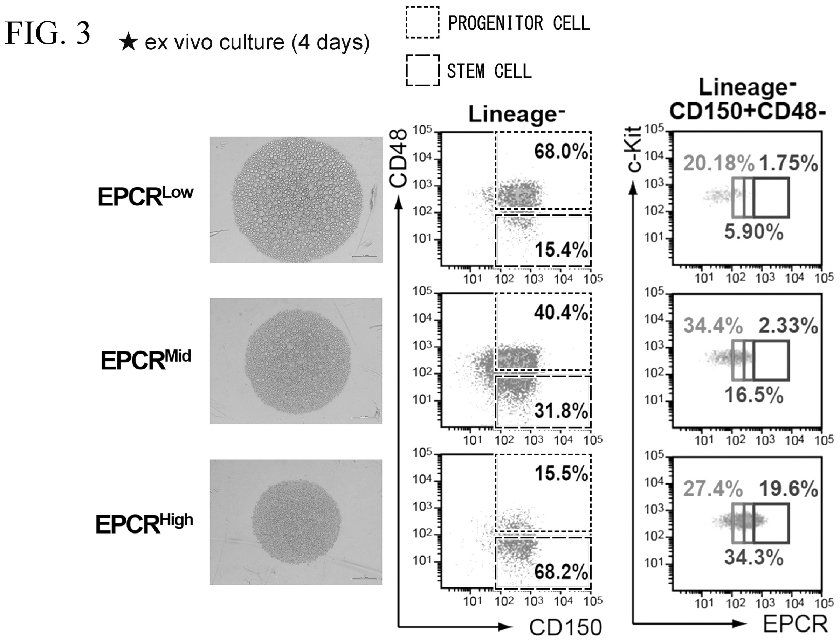

The EPCR High cell group and the EPCR Low cell group shown in FIG. 1, and the EPCR Mid cell group which was the remaining EPCR-positive cell group were each cultured ex vivo for 4 days, and then the expression patterns of CD150 and CD48 were checked by flow cytometry. As a result, it was confirmed that the EPCR High cell group maintains the phenotype (CD150+, CD48-) of stem cells even under the normal culture conditions, as compared with the EPCR Low cell group. Further, the CD150+ and CD48- cells were collected, and the proportion of the EPCR High cell group was quantified again by flow cytometry. As shown in FIG. 3, the proportions of the EPCR High in the EPCR Low cell group and the EPCR Mid cell group were each 1.75% and 2.33%. On the other hand, the proportion of the EPCR High cell group in the EPCR High was 19.6%. It was confirmed that the EPCR High cell group is hierarchically higher than the EPCR Low cell group and the like since the EPCR High cell group has a high ability to produce the EPCR High cell group exhibiting high stem cell properties as shown in FIG. 2, as compared with the EPCR Low cell group and the like.

The culture medium used for the ex vivo culture of the untreated hematopoietic stem cells isolated from the mouse is a culture medium obtained by adding, to the RPMI medium, L-Alanin (4.45mg/L), Zinc Sulfate Heptahydrate (0.432mg/L), Cupric Sulfate Pentahydrate (0.0013mg/L), Ferric Nitrate Nonahydrate (0.05mg/L), Ferrous Sulfate Heptahydrate (0.417mg/L), Linoleic acid (0.042mg/L), Hypoxanthine (2.1mg/L), Putrescine + 2HCL (0.081mg/L), Thioctic Acid (0.105mg/L), Thymidine (0.365mg/L), Sodium Pyruvate (55mg/L), HEPES (1mM), a penicillin-streptomycin mixed solution (Nacalai Tesque, Inc.: 26253-84) (1/100 capacity), Insulin-Transferrin-Selenium-Ethanolamine (Thermo Fisher Scientific, Inc.: 51500056) (1/100 capacity), and AlbuMAXtm I Lipid-Rich BSA (Thermo Fisher Scientific, Inc.: 11020021) (2%).

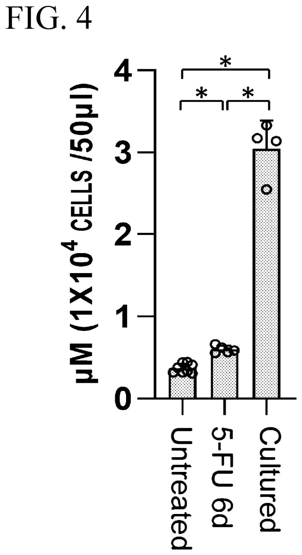

The glutamic acid levels of untreated hematopoietic stem cells isolated from a mouse, hematopoietic stem cells isolated from a mouse 6 days after the administration of 5-FU (250 mg/Kg), and hematopoietic stem cells subjected to the ex vivo culture were measured. The results are shown in FIG. 4. It was confirmed that the intracellular level of glutamic acid is low under conditions in which the cells are divided while maintaining the EPCR High phenotype after the 5-FU administration, as compared with conditions in which the EPCR High phenotype is lost by the ex vivo culture.

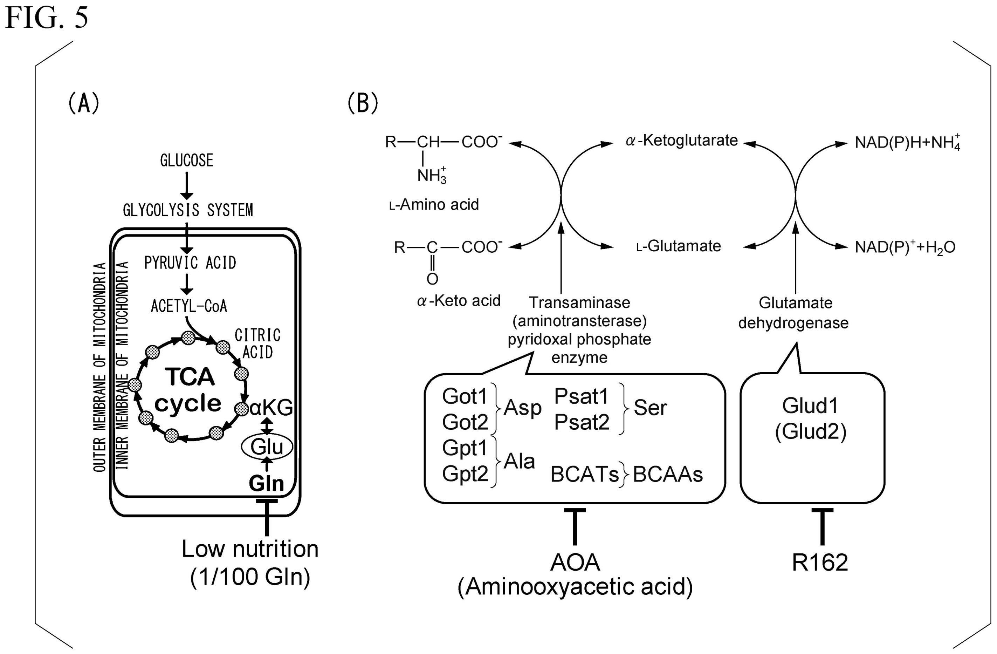

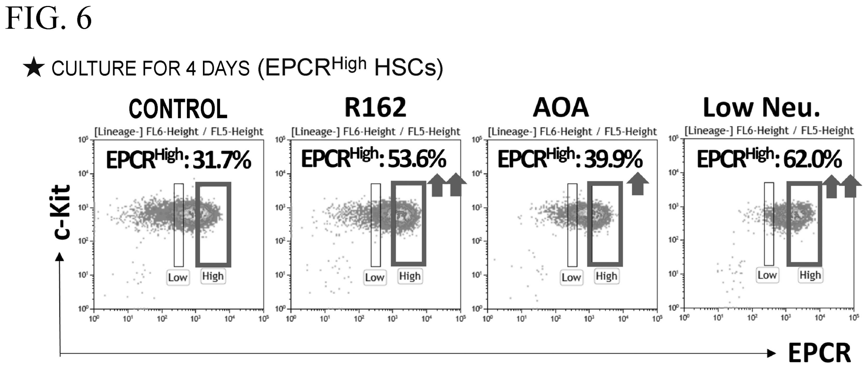

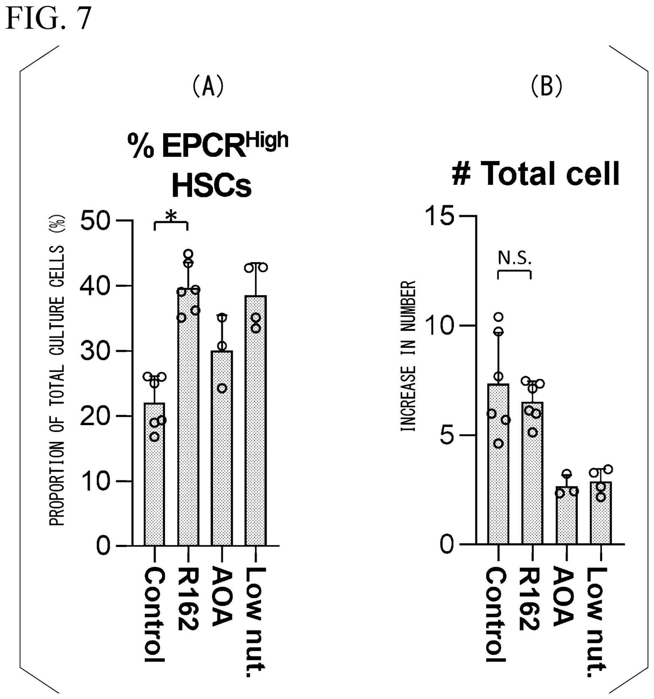

As shown in FIG. 5(A), α-ketoglutaric acid (αKG), which is an intermediate metabolite of the TCA cycle, is also produced from glutamic acid or the like, and then it is supplied to the TCA cycle. In addition, in the production of α-ketoglutaric acid from glutamic acid, two pathways of a transaminase pathway and a glutamate dehydrogenase pathway are used (see FIG. 5(B)). Therefore, the proportion of EPCR High in hematopoietic stem cells was determined in a case where the EPCR High cell group was cultured for 4 days by adding each of an inhibitor R162 (30 µM) of glutamate dehydrogenase and an inhibitor aminooxyacetic acid (AOA) (50 µM) of transaminase to the culture medium, and in a case where the content of glutamic acid in the culture medium, which is the source of glutamic acid synthesis, was set to 1/100 (0.02 mM) in FIG. 5(A) (Low Neu.). The results of the flow cytometry are shown in FIG. 6, and the quantification results thereof are shown in FIG. 7(A). It was confirmed that the phenotype of EPCR High is maintained by the inhibition of glutamate dehydrogenase. Further, as shown in FIG. 7(B), it was confirmed that in a case where AOA is added and in a case where the content of glutamic acid in the culture medium is 1/100, the number of cells is significantly reduced as compared with the control in a case where the cells are cultured for 4 days under each condition, whereas in a case where R162 is added, the number of cells is about the same level as that of the control, and the effect on cell proliferation is small. As a result, in a case of culturing in the presence of R162, the number of cells having the EPCR High phenotype is increased by about 2 times in the culture for 4 days, as compared with the culture under the normal culture conditions.

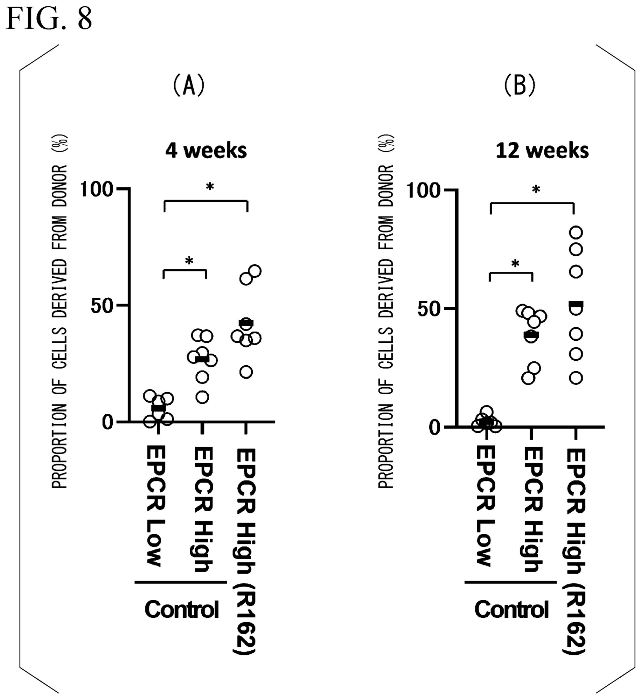

Mouse EPCR High cells were cultured for 4 days under the normal culture conditions or in the presence of R162, and 50 cells of each of an EPCR High cell group (Control) generated under the normal culture conditions, an EPCR Low cell group (Control), and an EPCR High cell group (R162) amplified in the presence of R162 were aliquoted and then transplanted into a recipient mouse. Then, after 4 weeks and 12 weeks, the proportion of cells derived from donor cells in the peripheral blood was examined. As shown in FIG. 8, it was confirmed that as compared with the EPCR Low cell group (Control), the EPCR High (R162) cell group amplified in the presence of R162 undergoes self-replication and division in the body of the host and has a high ability to reconstruct hematopoietic tissue, which is equivalent to the EPCR High cell group (Control) maintained under the normal culture conditions.

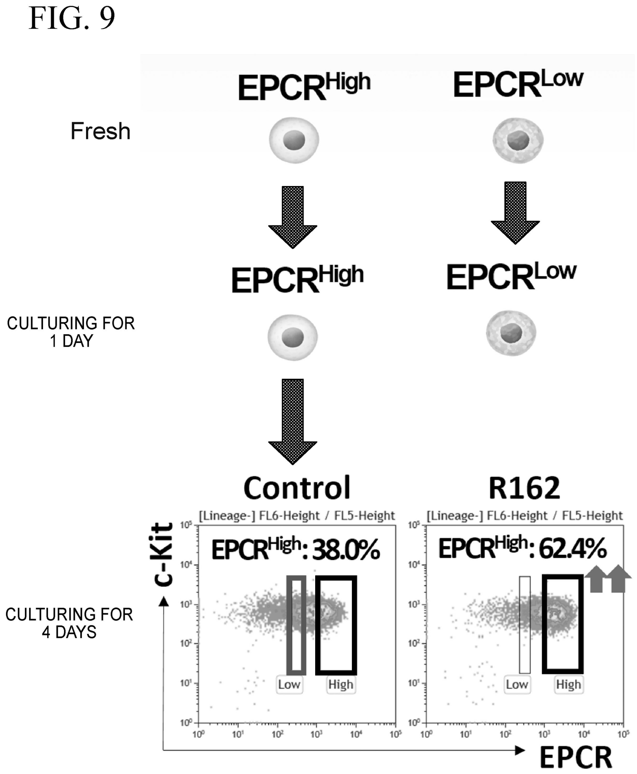

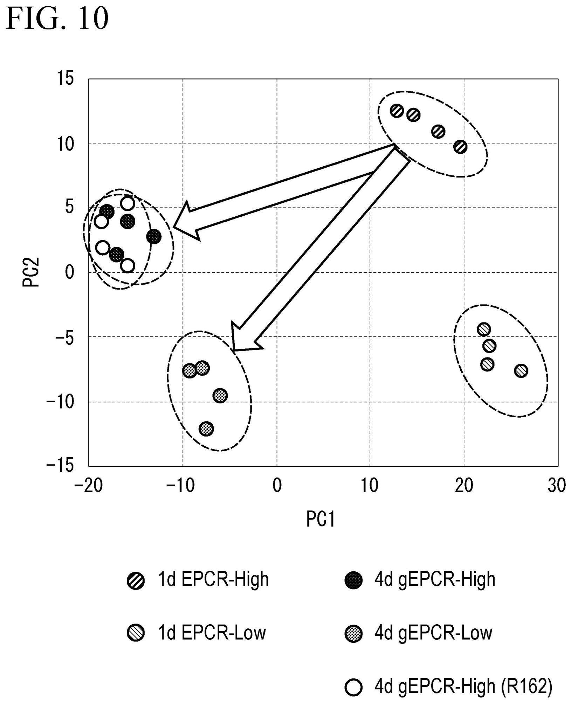

As shown in FIG. 9, the gene expression patterns were analyzed by RNA-sequence for the following cell groups: the EPCR High cell group (1d EPCR-High) cultured for 1 day; the EPCR Low cell group (1d EPCR-Low) cultured for 1 day; the EPCR High cell group (4d gEPCR-High) and the EPCR Low cell group (4d gEPCR-Low), which were obtained by separating again the EPCR High cell group after culturing for 4 days; and the EPCR High cell group (4d gEPCR-High (R162)) obtained by separating again the EPCR High cell group after culturing for 4 days in the presence of R162. As a result of the principal component analysis using the information on the expression of all genes, as shown in FIG. 10, the 4d gEPCR-High and 4d gEPCR-High (R162) which were cultured for 4 days exhibited extremely similar gene expression patterns.

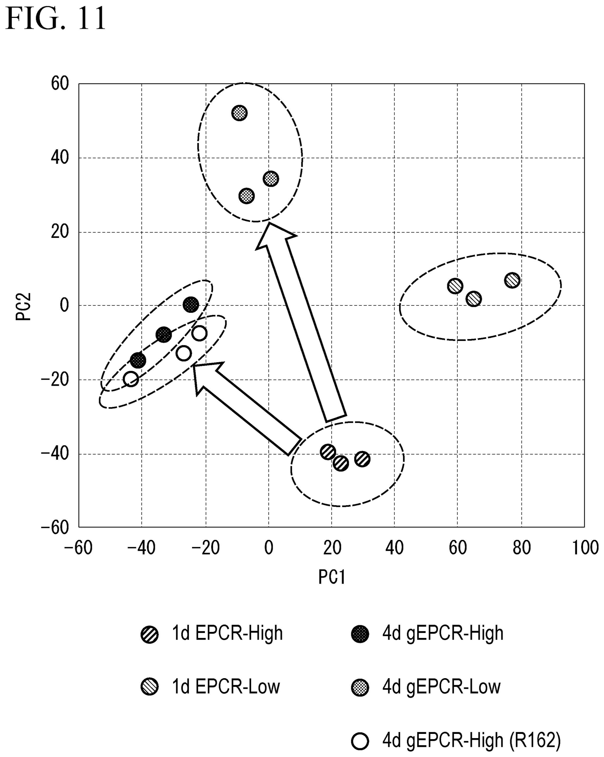

In the same manner, the chromatin status of each cell group was analyzed by open chromatin region analysis (ATAC-sequence). As a result of the principal component analysis on the chromatin accessibility of the region corresponding to the cis-element of each cell fraction, as shown in FIG. 11, the 4d gEPCR-High and 4d gEPCR-High (R162) which were cultured for 4 days exhibited extremely similar gene expression patterns even in the chromatin status.

The cells obtained by the expansion culture of the hematopoietic stem cells using R162 exhibited the stem cell activity, the gene expression pattern, and the chromatin status, which were comparable to those of the hematopoietic stem cells cultured under normal conditions.

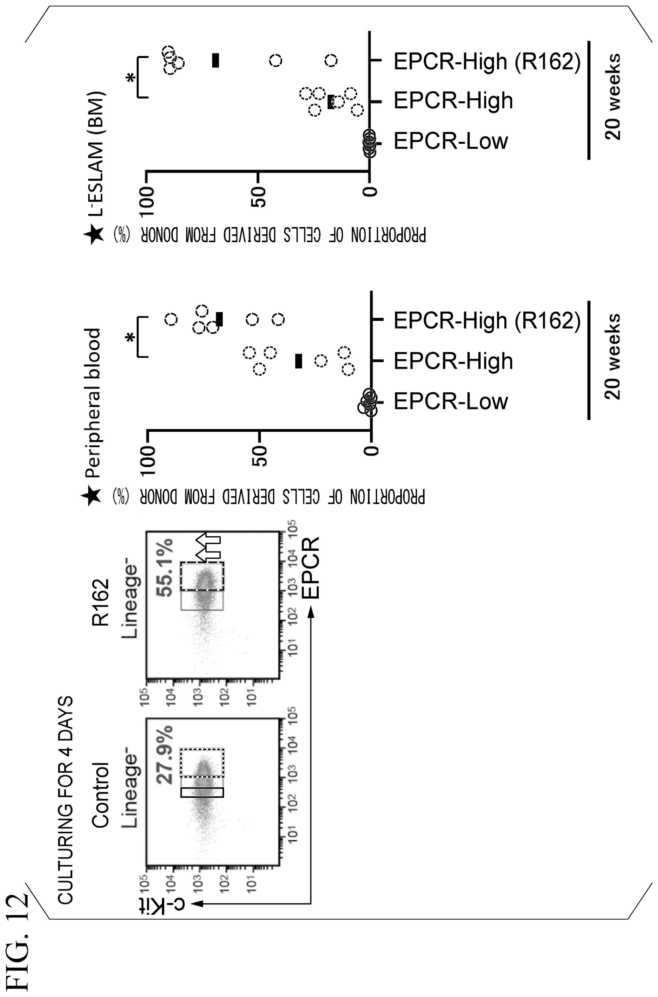

Further, the EPCR High cells were cultured for 4 days under the normal culture conditions and cultured for 4 days in the presence of R162, and the EPCR High cell group generated under the normal culture conditions, the EPCR Low cell group, and the EPCR High cell group (R162) amplified in the presence of R162 were each transplanted into a recipient mouse. Then, 20 weeks later, the proportion of cells derived from donor cells was examined in the peripheral blood and the hematopoietic stem cell fraction in the bone marrow (L-ESLAM (BM)). As shown in FIG. 12, it has been confirmed that the EPCR High (R162) cell group amplified in the presence of R162 can continue to survive for a long period of time after undergoing self-replication and division in the body of the host, and has a high ability to maintain the reconstructed hematopoiesis for a longer period of time, as compared with the EPCR High cell group and the EPCR Low cell group (Control), which are maintained under the normal culture conditions.

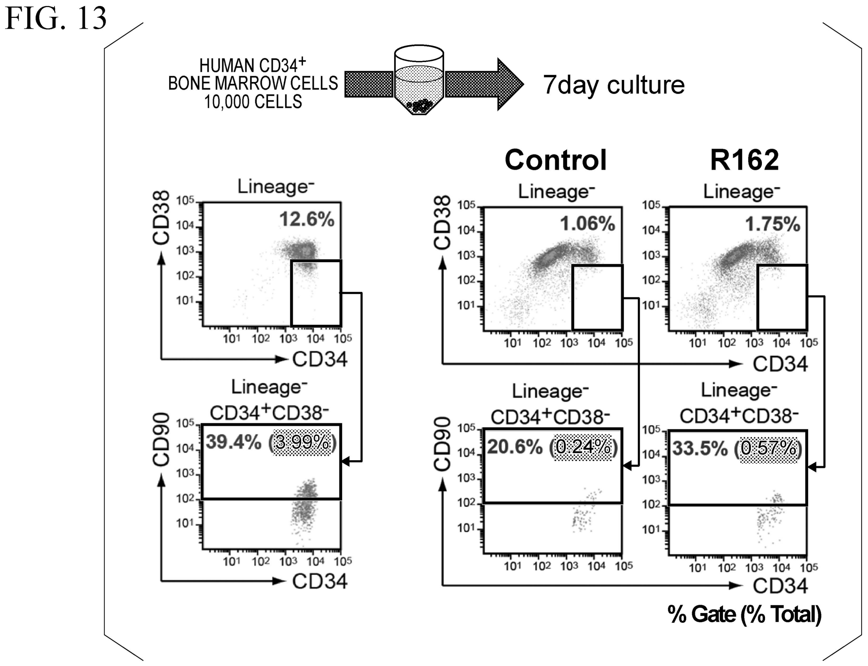

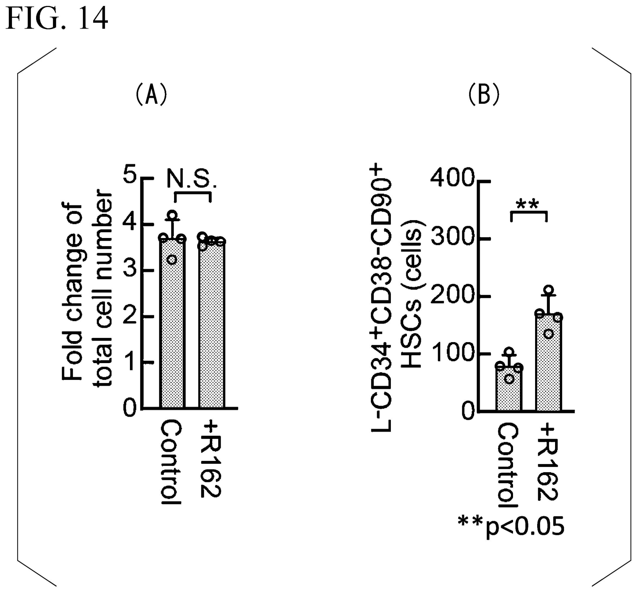

Human CD34⁺ bone marrow cells (10,000 cells) were cultured for 7 days under normal culture conditions or in the presence of a glutamate dehydrogenase inhibitor R162 (30 µM), and then the total number of cells was analyzed. Then, the proportion of hematopoietic stem cells (lineage: CD34+ CD38- CD90+ cells) was analyzed using a flow cytometer, and from the results, the number of stem cells was estimated. FIG. 13 and FIG. 14 show the results. As shown in FIG. 14(A), it has been confirmed that the total number of cells is not changed by the presence of R162 as compared with the control, whereas as shown in FIG. 14(B), the ratio of stem cells after culturing is increased by the presence of R162. It has been confirmed that as in the case of culturing mouse hematopoietic stem cells, R162 increases the ratio of stem cells after culturing without affecting the cell proliferation of the human hematopoietic stem cells, and thus, as a result, the number of stem cells is increased in a case of carrying out culturing in the presence of R162 as compared with a case of culturing under normal culture conditions.

The culture medium used for the ex vivo culture of human CD34+ bone marrow cells is a culture medium obtained by adding, to the RPMI medium, L-Alanin (4.45mg/L), Zinc Sulfate Heptahydrate (0.432mg/L), Cupric Sulfate Pentahydrate (0.0013mg/L), Ferric Nitrate Nonahydrate (0.05mg/L), Ferrous Sulfate Heptahydrate (0.417mg/L), Linoleic acid (0.042mg/L), Hypoxanthine (2.1mg/L), Putrescine + 2HCL (0.081mg/L), Thioctic Acid (0.105mg/L), Thymidine (0.365mg/L), Sodium Pyruvate (55mg/L), HEPES (1mM), a penicillin-streptomycin mixed solution (Nacalai Tesque, Inc.: 26253-84) (1/100 capacity), Insulin-Transferrin-Selenium-Ethanolamine (Thermo Fisher Scientific, Inc.: 51500056) (1/100 capacity), and AlbuMAXtm I Lipid-Rich BSA (Thermo Fisher Scientific, Inc.: 11020021) (2%).

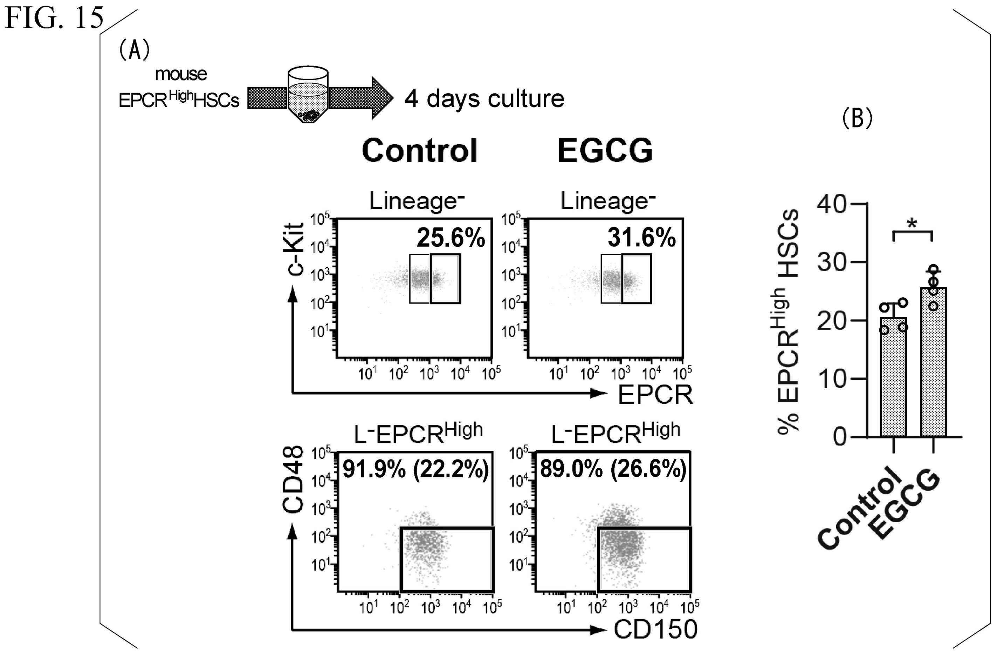

The above-described mouse EPCR High cell group was cultured for 4 days by adding EGCG (50 µM), which is another glutamate dehydrogenase inhibitor, to a culture medium. The results of the flow cytometry are shown in FIG. 15(A), and the quantification results thereof are shown in FIG. 15(B). It has been confirmed that the phenotype of EPCR High is maintained by the inhibition of glutamate dehydrogenase by EGCG.

### INDUSTRIAL APPLICABILITY

According to the present invention, hematopoietic stem cells can be subjected to expansion culture based on a new mechanism of action.

## Claims

1. A culture medium for expansion culture of a mammalian hematopoietic stem cell, the culture medium comprising:
a glutamate dehydrogenase inhibitor as an active ingredient.

2. The culture medium according to Claim 1,
wherein the glutamate dehydrogenase inhibitor is R162 or a derivative thereof.

3. A production method for a hematopoietic stem cell, in which a mammalian hematopoietic stem cell is subjected to expansion culture in a culture medium containing, as an active ingredient, a glutamate dehydrogenase inhibitor.

4. The production method according to Claim 3,
wherein the glutamate dehydrogenase inhibitor is R162 or a derivative thereof.

5. A cell preparation comprising:
a mammalian hematopoietic stem cell in which glutamate dehydrogenase is inactivated or deficient.

6. The cell preparation according to Claim 5,
wherein in the hematopoietic stem cell, EPCR or a hematopoietic stem cell marker molecule equivalent to the EPCR is highly expressed.
